# EUROPEAN PATENT APPLICATION

(11) **EP 3 640 327 A1**
(43) Date of publication of application: **22.04.2020**
(21) Application number: 18818513.6
(22) Date of filing: 15.06.2018
(51) Int. Cl.: C12N 7/01, A61K 35/763, A61K 48/00, A61P 35/00

(54) **RECOMBINANT HERPES SIMPLEX VIRUS, PREPARATION METHOD THEREFOR, AND APPLICATION THEREOF**

(30) Priority: 15.06.2017 CN 201710451582
(71) Applicant: Beijing Wellgene Company Ltd., Beijing 100085 (CN)
(72) Inventor: LI, Xiaopeng, Beijing 100085 (CN); TIAN, Chao, Beijing 100085 (CN); ZHAO, Jingshu, Beijing 100085 (CN); LIU, Jiajia, Beijing 100085 (CN); ZHOU, Hua, Beijing 100085 (CN); SUN, Chunyang, Beijing 100085 (CN)
(74) Representative: karo IP
(86) International application number: PCT/CN2018/091530
(87) International publication number: WO 2018/228538

(57) **Abstract**

Provided are a recombinant herpes simplex virus, a preparation method and use thereof. The recombinant herpes simplex virus comprises a vector and foreign genes encoding at least two cytokines, wherein the vector is a herpes simplex virus with genes encoding ICP34.5 and ICP47 deleted, and optionally with at least one of genes encoding ICP6, TK and UNG deleted, and the insertion site/sites of the foreign genes is in at least one of the positions where the genes encoding ICP34.5, ICP47, ICP6, TK and UNG are deleted in the vector.

## Description

### Technical Field

The present invention relates to the field of genetic engineering, in particular to a recombinant herpes simplex virus, a preparation method and use of the recombinant herpes simplex virus.

### Background Art

The traditional oncotherapy comprises surgery, radiotherapy and chemotherapy; however, the traditional oncotherapy usually has the defects of a low efficiency, severe side reactions and a high recurrence rate. Therefore, tumor immunotherapy has shown a good application prospect in recent years because of its advantages of a better therapeutic effect and less side reactions. A variety of immunotherapy methods have been approved for marketing and achieved a good therapeutic effect. In the Top 10 Scientific Breakthrough Ranking List in 2013 selected by the Science magazine, the tumor immunotherapy topped the list. Among them, the oncolytic virus played a great role in the tumor immunotherapy.

The oncolytic virus refers to a type of viruses which can selectively infect tumor cells and replicate in target cells, and ultimately lead to lysis and death of the tumor cells. Relying on their own specificity, this type of viruses replicate in the tumor cells to lyse the tumor cells. The viruses which are released after lysis of the cells can further infect surrounding tumor cells, and have no destructive effect or has less influence on normal cells and tissues. The oncolytic virus has multiple anti-tumor action mechanisms, comprising: 1) direct lysis of the tumor cells; 2) destruction of tumor blood vessels; 3) viral proteins produced by viral replication and expression having direct cytotoxicity; 4) anti-tumor immune response; and 5) enhanced sensitivity of the tumor cells to radiotherapy and chemotherapy. At present, the oncolytic viruses commonly used for anti-tumor researches comprise herpes simplex virus (HSV), adenovirus and vaccinia virus. However, due to the relatively complex genetic structure of the oncolytic viruses, and not thorough understanding of their properties, there are labile factors in the application. Moreover, due to the influence of the internal environment in the body, the ability of the oncolytic viruses to replicate in the body is often not satisfactory. These all seriously affect the anti-tumor effect of the oncolytic viruses.

Cytokines are signals that connect immune cells to each other, and regulate the response of the immune system to an antigen. The cytokines play an important role in the tumor immunotherapy, which can directly stimulate the expansion of immune effector cells at tumor sites and enhance the recognition of the tumor cells, and in the meantime activate the systematic immune response in the whole body. A large number of animal tumor models validate the anti-tumor activity of the cytokines. However, although the cytokines can be directly administrated for anti-tumor treatments, a large number of injections of the cytokines may bring side effects such as autoimmune diseases to the body.

Moreover, due to the multiple immune escape mechanisms in the tumor cells and the complexity of the internal environment in the body, no monotherapy strategy can eliminate the tumor cells very well. Therefore, it is very necessary to find a new anti-tumor method with a good anti-tumor effect and less side reactions.

### Summary of the Invention

The objective of the present invention is to provide a recombinant herpes simplex virus and a preparation method and use thereof to overcome the above defects existing in the prior art. The recombinant herpes simplex virus provided in the invention has a good anti-tumor effect and a small side reaction after use, and has a good application prospect.

In order to achieve the above objective, in a first aspect, the present invention provides a recombinant herpes simplex virus, wherein the recombinant herpes simplex virus comprises a vector and foreign genes encoding at least two cytokines, the vector being a herpes simplex virus with the genes encoding ICP34.5 and ICP47 deleted, and optionally with at least one of genes encoding ICP6, TK and UNG deleted, and the insertion site/sites of the foreign genes being in at least one of the positions where the genes encoding ICP34.5, ICP47, ICP6, TK and UNG are deleted in the vector.

In a second aspect, the present invention also provides a method for preparing the herpes simplex virus described above, comprising: knocking out the genes encoding ICP34.5 and ICP47 in the herpes simplex virus, and optionally knocking out at least one of the genes encoding ICP6, TK and UNG, and inserting the foreign genes encoding at least two cytokines, wherein the insertion site/sites of the foreign genes is/are in at least one of the positions where the genes encoding ICP34.5, ICP47, ICP6, TK and UNG are knocked out on the vector.

In a third aspect, the present invention also provides the use of the recombinant herpes simplex virus described above and/or the herpes simplex virus prepared by the above method in the preparation of a medicament for preventing and/or treating a tumor.

A stably expressed recombinant herpes simplex virus in the present invention is obtained by using a herpes simplex virus with the genes encoding ICP34.5 (infected cell polypeptide 34.5) and ICP47 deleted, and optionally with at least one of the genes encoding ICP6, TK (thymidine kinase) and UNG (uracil-N-glycosylase) deleted as a vector, and inserting foreign genes encoding at least two cytokines into the position/positions where the above-mentioned genes are deleted, particularly inserting the genes of at least two of GM-CSF, IL-2 and IL-12. Moreover, the recombinant herpes simplex virus exhibits a good anti-tumor effect, particularly for melanocytoma and breast cancer, and it can not only result in a targeting anti-tumor effect, but also has a small side reaction after use, by the way of local intratumoral injection; therefore, the recombinant herpes simplex virus has a good application prospect.

### Detailed Description of Embodiments

The endpoints of ranges and any values disclosed herein are not limited to the precise ranges or values, and these ranges or values should be construed as including the values that are close to these ranges or values. For numerical ranges, endpoint values of various ranges, an endpoint value of the various ranges and an individual point value, and the individual point values can be combined with each other to yield one or more new numerical ranges. These numerical ranges should be considered as specifically disclosed herein.

In a first aspect, the present invention provides a recombinant herpes simplex virus, wherein the recombinant herpes simplex virus comprises a vector and foreign genes encoding at least two cytokines, the vector being a herpes simplex virus with the genes encoding ICP34.5 and ICP47 deleted, and optionally with at least one of genes encoding ICP6, TK and UNG deleted, and the insertion site/sites of the foreign genes being in at least one of the positions where the genes encoding ICP34.5, ICP47, ICP6, TK and UNG are deleted in the vector.

It is desired to explain that the insertion site/sites of the foreign genes is/are in at least one of the positions where the genes are deleted. For example, if the genes encoding ICP34.5, ICP47, ICP6 and TK are deleted, the insertion site is any one position of the deletions other than the site of the gene encoding UNG. The at least two cytokines can be inserted into the same site after linked together or separately inserted into different sites. Preferably, the cytokines are inserted into the same site after linked together.

In the present invention, the genes encoding ICP34.5, ICP47, ICP6, TK, and UNG are well known to those skilled in the art, and can also be searched by logging into a relevant database. For example, the relevant nucleotide sequences can be searched by logging into the GenBank database, which is a conventional technical means available to those skilled in the art, and will not be repeated here in the present invention.

According to the present invention, the cytokines may be at least two of an interleukin (IL), a colony stimulating factor (CSF), an interferon (IFN), a tumor necrosis factor (TNF), a transforming growth factor, a growth factor and a chemokine, preferably at least two of GM-CSF, IL-2, IL-7, IL-12, IL-15, IL-18, IL-21, TNF-α, IFN-γ, IFN-α and IFN-β, and more preferably at least two of GM-CSF, IL-2 and IL-12.

In the present invention, the genes encoding the cytokines can be searched by logging into a relevant database. For example, the relevant nucleic acid sequences can be searched by logging into the GenBank database. In addition, the genes encoding the cytokines may be from different origin, depending on the subject to which the obtained recombinant herpes simplex virus is applied. For example, the genes encoding the cytokines may be from human when the obtained recombinant herpes simplex virus is applied to a human, and may be from mice when the obtained recombinant herpes simplex virus is applied to a mouse.

Specifically, when the obtained recombinant herpes simplex virus is applied to a human, the Gene IDs of the genes encoding GM-CSF, IL-2, IL-12A, IL-12B and TNF-α are 1437, 3558, 3592, 3593, and 7124, respectively.

When the obtained recombinant herpes simplex virus is applied to a mouse, the Gene IDs of the genes encoding GM-CSF, IL-2, IL-12A, IL-12B and TNF-α are 12981, 16183, 16159, 16160, and 21926, respectively.

According to the present invention, in order to make the genes encoding the cytokines efficiently and independently translated and expressed, the foreign gene of the present invention preferably further comprises a promoter, a start codon and a stop codon, and optionally a linker sequence and/or a PolyA sequence. In such a preferred case, when the recombinant herpes simplex virus infects a host cell and expresses its own genes, it is capable of transcribing independent and intact mRNA fragments of interest, thereby the genes of interest can be efficiently and independently translated.

In one preferred embodiment of the present invention, when the foreign genes encoding at least two cytokines are inserted into one position on the vector (for example, the position where the gene encoding ICP34.5 or the gene encoding ICP47 is deleted in the vector), in order to make the foreign gene encoding each of the cytokines individually expressed, a linker sequence should be included between the foreign gene encoding each of the cytokines. Preferably, the linker sequence is an IRES sequence.

In another preferred embodiment of the present invention, when the foreign genes encoding at least two cytokines are inserted into one position on the vector (for example, the position where the gene encoding ICP34.5 or the gene encoding ICP47 is deleted in the vector), in order to make the foreign gene encoding each of the cytokines individually expressed, the foreign gene encoding each of the cytokines has an independent expression cassette individually, and each of the independent expression cassettes has a promoter, a cytokine coding sequence, and a PolyA sequence.

According to the present invention, the type of the promoter is not particularly limited as long as the transcription of the foreign gene can be controlled. In a preferred case, the promoter is selected from at least one of a CMV promoter, an EF1α promoter, an SV40 promoter, an RSV promoter and an MMTV promoter, preferably a CMV promoter and/or an EF1α promoter.

In the present invention, the foreign gene may also comprise a marker gene (e.g., a gene encoding β-galactosidase, luciferase, green fluorescent protein or other fluorescent proteins). Moreover, the foreign gene may further comprise relevant transcriptional regulatory sequences typically associated with the sequence transcription, for example, a polyadenylation site, a Kozak sequence, a WPRE, and a downstream enhancer element. These are well known to those skilled in the art, and will not be repeated here in the present invention.

In the present invention, the type of the herpes simplex virus is not particularly limited and may be selected routinely in the art. However, the herpes simplex virus is preferably herpes simplex virus type I for the purpose of better achieving stable expression of cytokines. The source of the herpes simplex virus is also not particularly limited in the present invention, and the herpes simplex virus can be commercially available in a conventional manner or obtained by self-isolation in the laboratory.

The tumor treatment effect of the above recombinant herpes simplex virus of the present invention is significantly superior to that by using the recombinant herpes simplex virus into which only one cytokine is inserted alone. The therapeutic effect produced by the technical solution of inserting two or more cytokines is a synergistic effect.

In a second aspect, the present invention also provides a method for preparing a recombinant herpes simplex virus, comprising: knocking out the genes encoding ICP34.5 and ICP47 in the herpes simplex virus, and optionally knocking out at least one of the genes encoding ICP6, TK and UNG, and inserting the foreign genes encoding at least two cytokines, wherein the insertion site/sites of the foreign genes is/are in at least one of the positions where the genes encoding ICP34.5, ICP47, ICP6, TK and UNG are knocked out on the vector.

In the present invention, the knockout of the above genes encoding ICP, TK and UNG can be carried out by using various conventional methods in the art, and the present invention is not particularly limited thereto, for example, the knockout can be achieved by targeted knockout by means of homologous recombination, or alternatively, by targeted knockout by means of CRISPR. Under the premise of understanding the objective of the present invention and the viral vector used in the present invention, those skilled in the art can achieve the knockout of the gene encoding ICP according to routine technical means mastered by them.

In the present invention, the insertion of a foreign gene may also be carried out using various conventional methods in the art. The way of insertion may be directly inserting the gene of interest into a selected insertion site. For example, the insertion may be performed by means of CRISPR, or by means of homologous recombination so that a part of base sequences is replaced and the gene of interest is inserted. The latter is preferred in the present invention.

In the present invention, the recombinant herpes simplex virus also needs to complete its life history in a host cell, which is the same as the normal herpes simplex virus; therefore, the passage and propagation of the recombinant virus needs to be carried out in the host cell. The host cell may be various host cells that are capable of culturing the viral vector and/or recombinant virus of the present invention, for example, African green monkey kidney cells (Vero cells), hamster kidney cells (BHK cells), primary rabbit kidney cells, chick-embryo cells, amnion cells, human cervical cancer cells (Hela cells), and human embryonic lung diploid fibroblasts (WI-38 cells).

In addition, the present invention also provides a virus-infected cell, wherein the virus is the recombinant herpes simplex virus described above, and the cell has genes encoding and expressing ICP34.5 and ICP47, and optionally at least one of genes encoding and expressing ICP6, TK and UNG.

In the present invention, the particular selection of the cells can be made by reference to the selection of the host cells as enumerated above, and will not be repeated here.

In a third aspect, the present invention also provides the use of the recombinant herpes simplex virus described above and/or the recombinant herpes simplex virus prepared by the above method in the preparation of a medicament for preventing and/or treating a tumor. Preferably, the tumor is at least one of melanocytoma, brain glioma, head and neck tumor, liver cancer, ovarian cancer, prostate cancer, breast cancer, lung cancer, colorectal cancer, renal cell carcinoma, gastric cancer, pancreatic cancer, lymphoma and bladder cancer.

In a fourth aspect, the present invention relates to a genetically engineered herpes simplex virus, comprising deletions of ICP34.5 and ICP47 genes and a tandem introduction of at least two cytokine genes at the sites/site of the ICP34.5 and/or ICP47 gene deletion, wherein the introduced genes are selected from: GM-CSF, IL-2, IL-7, IL-12, IL-15, IL-18, IL-21, TNF-α, IFN-γ, IFN-α and IFN-β, and wherein the genetically engineered herpes simplex virus exhibits a synergistic oncolytic effect (tumor treatment effect). The term "synergistic" as used in the present invention means that the oncolytic effect (tumor treatment effect) exhibited by the genetically engineered herpes simplex virus into which two or more cytokines promoting (enhancing) the immune response have been tandemly introduced is greater than that exhibited by the genetically engineered herpes simplex viruses into which each of the cytokines promoting the immune response is separately introduced.

The "synergy" of the oncolytic effect or the tumor treatment effect described in the present invention is demonstrated by the fact that the effect achieved by tandemly introducing the cytokines enhancing the immunization is greater than the sum of the effects resulted from separately introducing the cytokines. The synergistic effect exhibited by the recombinant herpes simplex virus or the genetically engineered herpes simplex virus of the present invention is determined according to the method used in the examples of the present application. Other determination methods in the art can also be used, for example, the combination method and the dose-effect analysis method described by Chou and Talalay (Chou and Talalay (1984) Adv. Enzyme Regul. 22: 27-55; Chou and Talalay, "New Avenues in Developmental Cancer Chemotherapy", Academic Press, 1987, Chapter 2) are used for determination.

The "tandem" introduction in the technical solution of the present invention means that the two or more cytokines, e.g., the cytokines that promote (enhance) the immune response are introduced into the same gene deletion site of the herpes simplex virus, the deletion site being selected from one of ICP34.5, ICP47, ICP6, TK and UNG; alternatively, the two or more cytokines, e.g., the cytokines that promote (enhance) the immune response, are introduced into different gene deletion sites of the herpes simplex virus, the deletion sites being selected from ICP34.5, ICP47, ICP6, TK and UNG. By tandem introduction, the genetically engineered herpes simplex virus of the present invention simultaneously carries two or more, preferably two, three or four genes encoding the cytokines that promote (enhance) the immune response.

In one preferred embodiment of the present invention, two, three or four of the cytokines selected from GM-CSF, IL-2, IL-12, TNF-α and IFN-γ, are introduced into the recombinant herpes simplex virus or the genetically engineered herpes simplex virus.

In a preferred embodiment, the herpes simplex virus described above is herpes simplex virus type I.

In a fifth aspect, the present invention also relates to a stock solution, and a host cell as well as a pharmaceutical composition comprising the recombinant herpes simplex virus or the genetically engineered herpes simplex virus described above. Meanwhile, the present invention also relates to the use of the stock solution, and the host cell as well as the pharmaceutical composition comprising the recombinant herpes simplex virus or the genetically engineered herpes simplex virus described above in the preparation of a medicament for treating a tumor, especially a solid tumor, preferably melanocytoma, brain glioma, head and neck tumor, liver cancer, lung cancer, colorectal cancer, renal cell carcinoma, gastric cancer, pancreatic cancer, lymphoma, bladder cancer, ovarian cancer, prostate cancer, and breast cancer.

In a sixth aspect, the present invention also relates to a method for treating a tumor, especially a solid tumor, preferably melanocytoma, brain glioma, head and neck tumor, liver cancer, lung cancer, colorectal cancer, renal cell carcinoma, gastric cancer, pancreatic cancer, lymphoma, bladder cancer, ovarian cancer, prostate cancer, and breast cancer, the method comprising administering to a subject an effective amount of the recombinant herpes simplex virus or the genetically engineered herpes simplex virus described above. In a preferred embodiment, the herpes simplex virus of the present invention is topically administered to a tumor for treatment, and the treatment is performed by introducing the herpes simplex virus into the tumor tissues (intratumoral administration), preferably via a catheter or injection.

The present invention will be described in detail below by way of examples.

In the following examples and comparative examples:

Vero cells were purchased from ATCC under the Cat# CCL-81;
the artificially chemical synthesis of the foreign genes was carried out by Genewiz (Suzhou) Biotechnology Co., Ltd. (Jiangsu, China);
the B16-BL6 cells and the 4T1 cells were obtained from the Academy of Military Medical Sciences (Beijing, China), the H22 cells were obtained from the cell bank of the China Center for Type Culture Collection, and the Balb/c mice were obtained from the Academy of Military Medical Sciences and Beijing Vital River Laboratory Animal Technology Co., Ltd. (Beijing, China).

### Example 1

This example is intended to illustrate the construction of a recombinant herpes simplex virus provided by the present invention.

According to the method described in the patent application with the application number 2004100064921 and the allowed patent number CN1283803C, the ICP34.5 gene and the ICP47 gene of the wild-type HSV-1 virus (the gene sequence number in GenBank is NC_001806, the same below) were knocked out, and the artificially chemically synthesized foreign genes were inserted into the position of the HSV-1 virus where the ICP34.5 gene was knocked out, except that the Vero cells were used as host cells in this example. The foreign genes comprise in order from 5' end to 3' end: a CMV promoter, a gene encoding GM-CSF (Gene ID: 12981), a BGH PolyA, an EF1α promoter, a gene encoding IL-2 (Gene ID: 16183) and a TK PolyA. The herpes simplex virus vector into which the GM-CSF and IL-2 coding genes had been correctly inserted was identified by sequencing in Beijing Sunbiotech Co. Ltd., thereby obtaining the recombinant viral vector. The successfully constructed recombinant viral vectors were propagated in Vero host cells at 37°C and 5% CO₂, with an infection multiplicity of 0.1. After harvesting, the cell debris was removed with a 0.65 µm filter, then the viral vectors were purified by a high-speed centrifugation at 13,000 rpm. The virus suspension with a titer of 1 × 10⁸ pfu/mL was obtained for use in animal experiments.

### Example 2

This example is intended to illustrate the construction of a recombinant herpes simplex virus provided by the present invention.

The recombinant herpes simplex virus was constructed according to the method of Example 1, except that the inserted foreign genes comprise in order from 5' end to 3' end: a CMV promoter, a gene encoding GM-CSF (Gene ID: 12981), a BGH PolyA, an EF1α promoter, a gene encoding IL-12B (Gene ID: 16160), an IRES sequence, a gene encoding IL-12A (Gene ID: 16159) and a TK PolyA.

### Example 3

This example is intended to illustrate the construction of a recombinant herpes simplex virus provided by the present invention.

According to the method described in the patent application with the application number 2004100064921 and the allowed patent number CN1283803C, the ICP34.5 gene and the ICP47 gene of the wild-type HSV-1 virus were knocked out, and the artificially chemically synthesized foreign gene 1 was inserted into the position of the HSV-1 virus where the ICP34.5 gene was knocked out, and the artificially chemically synthesized foreign gene 2 was inserted into the position of the HSV-1 virus where the ICP47 gene was knocked out, except that the Vero cells were used as host cells in this example. The foreign gene 1 comprises in order from 5' end to 3' end: an EF1α promoter, a gene encoding IL-2 (Gene ID: 16183) and a TK PolyA. The foreign gene 2 comprises in order from 5' end to 3' end: a CMV promoter, a gene encoding GM-CSF (Gene ID: 12981), a BGH PolyA. The herpes simplex virus vector into which the GM-CSF and IL-2 coding genes had been correctly inserted was identified by sequencing in Beijing Sunbiotech Co. Ltd., thereby obtaining the recombinant viral vector. The successfully constructed recombinant viral vectors were propagated in Vero host cells at 37°C and 5% CO₂, with an infection multiplicity of 0.1. After harvesting, the cell debris was removed with a 0.65 µm filter, then the viral vectors were purified by a high-speed centrifugation at 13,000 rpm. The virus suspension at 1 × 10⁸ pfu/mL was obtained for use in animal experiments.

### Example 4

This example is intended to illustrate the construction of a recombinant herpes simplex virus provided by the present invention.

According to the method described in the patent application with the application number 2004100064921 and the allowed patent number CN1283803C, the ICP34.5 gene and the ICP47 gene of the wild-type HSV-1 virus were knocked out, and the artificially chemically synthesized foreign gene 1 was inserted into the position of the HSV-1 virus where the ICP34.5 gene was knocked out, and the artificially chemically synthesized foreign gene 2 was inserted into the position of the HSV-1 virus where the ICP47 gene was knocked out. The foreign gene 1 comprises in order from 5' end to 3' end: an EF1α promoter, a gene encoding IL-12B (Gene ID: 16160), an IRES sequence, a gene encoding IL-12A (Gene ID: 16159), and a TK PolyA. The foreign gene 2 comprises in order from 5' end to 3' end: a CMV promoter, a gene encoding GM-CSF (Gene ID: 12981), and a BGH PolyA. The herpes simplex virus vector into which the GM-CSF, IL-12A and IL-12B coding genes had been correctly inserted was identified by sequencing in Beijing Sunbiotech Co. Ltd., thereby obtaining the recombinant viral vector. The successfully constructed recombinant viral vectors were propagated in Vero host cells at 37°C and 5% CO₂, with an infection multiplicity of 0.1. After harvesting, the cell debris was removed with a 0.65 µm filter, then the viral vectors were purified by a high-speed centrifugation at 13,000 rpm. The virus suspension at 1 × 10⁸ pfu/mL was obtained for use in animal experiments.

### Example 5

This example is intended to illustrate the construction of a recombinant herpes simplex virus provided by the present invention.

The recombinant herpes simplex virus was constructed according to the method of Example 1, except that the ICP34.5 gene, the ICP47 gene and the ICP6 gene of the wild-type HSV-1 virus were knocked out to obtain the recombinant viral vector.

### Example 6

This example is intended to illustrate the construction of a recombinant herpes simplex virus provided by the present invention.

The recombinant herpes simplex virus was constructed according to the method of Example 1, except that the insertion site of the foreign genes was the position of the HSV-1 virus where the ICP47 gene was knocked out.

### Example 7

This example is intended to illustrate the construction of a recombinant herpes simplex virus provided by the present invention.

The recombinant herpes simplex virus was constructed according to the method of Example 1, except that the inserted foreign genes comprise in order from 5' end to 3' end: a CMV promoter, a gene encoding GM-CSF (Gene ID: 12981), a BGH PolyA, an EF1α promoter, a gene encoding TNF-α (Gene ID: 21926), and a TK PolyA.

### Example 8

This example is intended to illustrate the construction of a recombinant herpes simplex virus provided by the present invention.

According to the method described in the patent application with the application number 2004100064921 and the allowed patent number CN1283803C, the ICP34.5 gene and the ICP47 gene of the wild-type HSV-1 virus were knocked out, and the artificially chemically synthesized foreign gene 1 was inserted into the position of the HSV-1 virus where the ICP34.5 gene was knocked out, and the artificially chemically synthesized foreign gene 2 was inserted into the position of the HSV-1 virus where the ICP47 gene was knocked out. The foreign gene 1 comprises in order from 5' end to 3' end: an EF1α promoter, a gene encoding IL-12B (Gene ID: 16160), an IRES sequence, a gene encoding IL-12A (Gene ID: 16159), and a TK PolyA. The foreign gene 2 comprises in order from 5' end to 3' end: a CMV promoter, a gene encoding TNF-α (Gene ID: 21926), and a BGH PolyA. The herpes simplex virus vector into which the IL-12A, IL-12B and TNF-α coding genes had been correctly inserted was identified by sequencing in Beijing Sunbiotech Co. Ltd., thereby obtaining the recombinant viral vector. The successfully constructed recombinant viral vectors were propagated in Vero host cells at 37°C and 5% CO₂, with an infection multiplicity of 0.1. After harvesting, the cell debris was removed with a 0.65 µm filter, then the viral vectors were purified by a high-speed centrifugation at 13,000 rpm. The virus suspension at 1 × 10⁸ pfu/mL was obtained for use in animal experiments.

### Comparative Example 1

The recombinant herpes simplex virus was constructed according to the method of Example 1, except that the inserted foreign gene comprises in order from 5' end to 3' end: a CMV promoter, a gene encoding GM-CSF (Gene ID: 12981), and a BGH PolyA.

### Comparative Example 2

The recombinant herpes simplex virus was constructed according to the method of Example 1, except that the inserted foreign gene comprises in order from 5' end to 3' end: an EF1α promoter, a gene encoding IL-2 (Gene ID: 16183), and a TK PolyA.

### Comparative Example 3

The recombinant herpes simplex virus was constructed according to the method of Example 1, except that the inserted foreign genes comprise in order from 5' end to 3' end: an EF1α promoter, a gene encoding IL-12B (Gene ID: 16160), an IRES sequence, a gene encoding IL-12A (Gene ID: 16159), and a TK PolyA.

### Comparative Example 4

The recombinant herpes simplex virus was constructed according to the method of Example 3, except that the ICP47 gene and the ICP6 gene of the wild-type HSV-1 virus were knocked out, and the artificially chemically synthesized foreign gene 1 was inserted into the position of the HSV-1 virus where the ICP6 gene was knocked out, and the artificially chemically synthesized foreign gene 2 was inserted into the position of the HSV-1 virus where the ICP47 gene was knocked out.

### Comparative Example 5

The recombinant herpes simplex virus was constructed according to the method of Example 1, except that only the ICP34.5 gene and the ICP47 gene of the wild-type HSV-1 virus were knocked out and no foreign gene was inserted.

### Comparative Example 6

The recombinant herpes simplex virus was constructed according to the method of Example 1, except that the inserted foreign gene comprises in order from 5' end to 3' end: a CMV promoter, a gene encoding TNF-α (Gene ID: 21926), and a BGH PolyA.

### Test Example 1

The B16-BL6 cells were inoculated in the Balb/c mice to establish a melanoma mouse model. The experimental mice in the successfully established model were randomly divided into experimental groups 1-14 and a control group. The virus suspensions obtained in Examples 1-8 and Comparative Examples 1-6 were administered to the experimental groups 1-14 (10 mice per group), respectively. Each group of experimental mice were intratumorally injected with the virus suspension once every 3 days for a total of 5 injections, 100 µL each time, and the mice in the control group were injected with 100 µL of PBS. After 14 days of administration, the therapeutic effect was evaluated by the relative tumor growth inhibition rate and tumor delay time. The tumor volume was calculated by the formula: long diameter × short diameter²/2, the relative tumor volume = the animal tumor volume after treatment / animal tumor volume before treatment, and the relative tumor growth inhibition rate TGI% = (1-T/C) × 100%. T/C % is the relative tumor proliferation rate, which is the relative tumor volume percentage value of the treatment group to the control group. T and C are the relative tumor volumes of the treatment group and the control group, respectively. The experimental results showed that the herpes simplex virus of the present invention exhibits a synergistic effect on the relative tumor growth inhibition rate, and the specific data is shown in Table 1.

**Table 1:**

| Group | TGI% |
|---|---|
| Control group (PBS) | --- |
| Experimental group 1 (Example 1) | 81 |
| Experimental group 2 (Example 2) | 85 |
| Experimental group 3 (Example 3) | 76 |
| Experimental group 4 (Example 4) | 78 |
| Experimental group 5 (Example 5) | 71 |
| Experimental group 6 (Example 6) | 73 |
| Experimental group 7 (Example 7) | 69 |
| Experimental group 8 (Example 8) | 47 |
| Experimental group 9 (Comparative Example 1) | 63 |
| Experimental group 10 (Comparative Example 2) | 55 |
| Experimental group 11 (Comparative Example 3) | 60 |
| Experimental group 12 (Comparative Example 4) | 67 |
| Experimental group 13 (Comparative Example 5) | 49 |
| Experimental group 14 (Comparative Example 6) | 52 |

| | |
|---|---|
| Note: "---" indicates that there is no any tumor growth inhibition effect, so the relative tumor growth inhibition rate cannot be obtained. | |

This experiment determines the effect of the herpes simplex virus of the present invention on the tumor delay time (T-C). The tumor delay time (T-C) refers to the delay days in the treatment group as compared with the control group when the tumor grew to 1200 mm³. T and C are the days required for the average tumor volume to reach 1200 mm³ in the treatment group and in the control group, respectively. The larger the T-C value is, the longer the delay time is, indicating that the efficacy is better; and vice versa. The experimental results showed that the herpes simplex virus of the present invention exhibits a synergistic effect on the tumor delay time (T-C), and the specific data is shown in Table 2.

**Table 2:**

| Group | **T-C (days)** |
|---|---|
| Control group (PBS) | --- |
| Experimental group 1 (Example 1) | 10.5 |
| Experimental group 2 (Example 2) | 13 |
| Experimental group 3 (Example 3) | 8 |
| Experimental group 4 (Example 4) | 9 |
| Experimental group 5 (Example 5) | 5 |
| Experimental group 6 (Example 6) | 5 |
| Experimental group 7 (Example 7) | 4.5 |
| Experimental group 8 (Example 8) | 3 |
| Experimental group 9 (Comparative Example 1) | 4.5 |
| Experimental group 10 (Comparative Example 2) | 4 |
| Experimental group 11 (Comparative Example 3) | 4 |
| Experimental group 12 (Comparative Example 4) | 4.5 |
| Experimental group 13 (Comparative Example 5) | 3.5 |
| Experimental group 14 (Comparative Example 6) | 4 |

### Assessment of the side effect:

During the experiment, the experimental animals in the experimental groups 1-8 had a good mental state, which is not significantly different from that of the experimental animals in the comparative examples, indicating that the herpes simplex virus of the present invention into which the cytokines are tandemly introduced has a significantly enhanced oncolytic effect, meanwhile the side effect was not increased with the increase of cytokine introduction.

### Test Example 2

The 4T1 cells were inoculated in the Balb/c mice to establish a breast cancer mouse model. The method for assessing the tumor treatment was the same as that in Test Example 1. The virus suspensions obtained in Examples 1-8 and Comparative Examples 1-6 were injected intratumorally, and the therapeutic effect was evaluated by the relative tumor growth inhibition rate (the calculation formula was the same as that in Test Example 1) 14 days after administration. The experimental results showed that the herpes simplex virus of the present invention exhibits a synergistic effect on the relative tumor growth inhibition rate, and the specific data is shown in Table 3.

**Table 3:**

| Group | TGI% |
|---|---|
| Control group (PBS) | --- |
| Experimental group 1 (Example 1) | 83 |
| Experimental group 2 (Example 2) | 89 |
| Experimental group 3 (Example 3) | 80 |
| Experimental group 4 (Example 4) | 78 |
| Experimental group 5 (Example 5) | 73 |
| Experimental group 6 (Example 6) | 73 |
| Experimental group 7 (Example 7) | 68 |
| Experimental group 8 (Example 8) | 51 |
| Experimental group 9 (Comparative Example 1) | 69 |
| Experimental group 10 (Comparative Example 2) | 58 |
| Experimental group 11 (Comparative Example 3) | 64 |
| Experimental group 12 (Comparative Example 4) | 61 |
| Experimental group 13 (Comparative Example 5) | 42 |
| Experimental group 14 (Comparative Example 6) | 55 |

| | |
|---|---|
| Note: "---" indicates that there is no any tumor growth inhibition effect, so the relative tumor growth inhibition rate cannot be obtained. | |

This experiment determines the effect of the herpes simplex virus of the present invention on the tumor delay time (T-C). The tumor delay time (T-C) refers to the delay days in the treatment group as compared with the control group when the tumor grew to 1200 mm³. T and C are the days required for the average tumor volume to reach 1200 mm³ in the treatment group and in the control group, respectively. The larger the T-C value is, the longer the delay time is, indicating that the efficacy is better; and vice versa. The experimental results showed that the herpes simplex virus of the present invention exhibits a synergistic effect on the tumor delay time (T-C), and the specific data is shown in Table 4.

**Table 4:**

| Group | **T-C (days)** |
|---|---|
| Control group (PBS) | --- |
| Experimental group 1 (Example 1) | 13 |
| Experimental group 2 (Example 2) | 15 |
| Experimental group 3 (Example 3) | 11.5 |
| Experimental group 4 (Example 4) | 10 |
| Experimental group 5 (Example 5) | 7 |
| Experimental group 6 (Example 6) | 6.5 |
| Experimental group 7 (Example 7) | 5.5 |
| Experimental group 8 (Example 8) | 3.5 |
| Experimental group 9 (Comparative Example 1) | 5 |
| Experimental group 10 (Comparative Example 2) | 4 |
| Experimental group 11 (Comparative Example3) | 5.5 |
| Experimental group 12 (Comparative Example 4) | 4.5 |
| Experimental group 13 (Comparative Example 5) | 3.5 |
| Experimental group 14 (Comparative Example 6) | 4 |

### Assessment of the side effect:

During the experiment, the experimental animals in the experimental groups 1-8 had a good mental state, which is not significantly different from that of the experimental animals in the comparative examples, indicating that the herpes simplex virus of the present invention into which the cytokines are tandemly introduced has a significantly enhanced oncolytic effect, meanwhile the side effect was not increased with the increase of cytokine introduction.

### Test Example 3

The H22 cells were intraperitoneally injected into the Balb/c mice after resuscitation. One week later, ascites was drawn for inoculation to establish a liver cancer mouse model. The method for assessing the tumor treatment was the same as that in Test Example 1. The virus suspensions obtained in Example 1, Example 4 and Comparative Examples 1-3 were injected intratumorally, and the therapeutic effect was evaluated by the relative tumor growth inhibition rate (the calculation formula was the same as that in Test Example 1) 14 days after administration. The experimental results showed that the herpes simplex virus of the present invention exhibits a synergistic effect on the relative tumor growth inhibition rate, and the specific data is shown in Table 5.

**Table 5:**

| Group | TGI% |
|---|---|
| Control group (PBS) | --- |
| Experimental group 1 (Example 1) | 79 |
| Experimental group 4 (Example 4) | 77 |
| Experimental group 9 (Comparative Example 1) | 58 |
| Experimental group 10 (Comparative Example 2) | 57 |
| Experimental group 11 (Comparative Example 3) | 54 |

It can be seen from the above-mentioned contents that the tumor treatment effects of the Example groups were obviously superior to those of the Comparative Examples, showing a synergistic effect.

### Assessment of the side effect:

During the experiments, the experimental animals in the above-mentioned experimental groups 1 and 4 had a good mental state, which is not significantly different from that of the experimental animals in the comparative examples, indicating that the herpes simplex virus of the present invention into which the cytokines are tandemly introduced has a significantly enhanced oncolytic effect, meanwhile the side effect was not increased with the increase of cytokine introduction.

By comparing the results of the Examples 1-8 with those of the Comparative Examples 1-6 described above, it can be known that the herpes simplex virus provided in the present invention exhibits a good anti-tumor effect, particularly for melanocytoma, breast cancer and liver cancer, and it can not only result in a targeting anti-tumor effect, but also has no obvious side reaction after use, by the way of local intratumoral injection; therefore, the herpes simplex virus in the present invention has a good application prospect.

The preferred embodiments of the present invention have been described in detail above, but the present invention is not limited thereto. Within the scope of the technical conception of the present invention, various simple variations can be made to the technical solutions of the present invention, comprising combinations of various technical features in any other suitable manner. These simple variations and combinations should also be regarded as the disclosure of the present invention and fall within the scope of protection of the present invention.

## Claims

1. A recombinant herpes simplex virus, wherein the recombinant herpes simplex virus comprises a vector and foreign genes encoding at least two cytokines, wherein the vector is a herpes simplex virus with genes encoding ICP34.5 and ICP47 deleted, and optionally with at least one of genes encoding ICP6, TK and UNG deleted, and the insertion site of the foreign genes is in at least one of the positions where the genes encoding ICP34.5, ICP47, ICP6, TK and UNG are deleted in the vector.

2. The recombinant herpes simplex virus according to claim 1, wherein the vector is a herpes simplex virus with the genes encoding ICP34.5 and ICP47 deleted, and optionally with the gene encoding ICP6 deleted, and the insertion site of the foreign gene is in the positions where the genes encoding ICP34.5 and/or ICP47 are deleted in the vector.

3. The recombinant herpes simplex virus according to claim 1, wherein the cytokines are at least two selected from an interleukin, a colony stimulating factor, an interferon, a tumor necrosis factor, a transforming growth factor, a growth factor and a chemokine, preferably at least two selected from GM-CSF, IL-2, IL-7, IL-12, IL-15, IL-18, IL-21, TNF-α, IFN-γ, IFN-α, and IFN-β, more preferably at least two selected from GM-CSF, IL-2 and IL-12.

4. The recombinant herpes simplex virus according to claim 1, wherein the foreign gene further comprises a promoter, a start codon and a stop codon, and optionally a linker sequence.

5. The recombinant herpes simplex virus according to claim 3, wherein the promoter is at least one selected from a CMV promoter, an EF1α promoter, an SV40 promoter, an RSV promoter and an MMTV promoter, preferably a CMV promoter and/or an EF1α promoter.

6. The recombinant herpes simplex virus according to claim 1, wherein the herpes simplex virus is herpes simplex virus type I.

7. A method for preparing the herpes simplex virus according to any one of claims 1-6, wherein the method comprises: knocking out the genes encoding ICP34.5 and ICP47 in the herpes simplex virus, and optionally knocking out at least one of the genes encoding ICP6, TK and UNG, and inserting foreign genes encoding at least two cytokines, wherein the insertion site of the foreign genes is in at least one of the positions where the genes encoding ICP34.5, ICP47, ICP6, TK and UNG are knocked out in the vector.

8. A use of the recombinant herpes simplex virus according to any one of claims 1-6 or the recombinant herpes simplex virus prepared by the method according to claim 7 in the preparation of a medicament for preventing and/or treating a tumor.

9. The use according to claim 8, wherein the tumor is at least one selected from melanocytoma, brain glioma, head and neck tumor, liver cancer, lung cancer, colorectal cancer, renal cell carcinoma, gastric cancer, pancreatic cancer, lymphoma, bladder cancer, ovarian cancer, prostate cancer, and breast cancer.

10. A genetically engineered herpes simplex virus, comprising deletions of ICP34.5 and ICP47 genes and a tandem introduction of at least two cytokine genes at the sites/site of the ICP34.5 and/or ICP47 gene deletion, wherein the introduced genes are selected from: GM-CSF, IL-2, IL-7, IL-12, IL-15, IL-18, IL-21, TNF-α, IFN-γ, IFN-α and IFN-β, and wherein the genetically engineered herpes simplex virus exhibits a synergistic oncolytic effect.

11. The genetically engineered herpes simplex virus of claim 10, further comprising a deletion of one or more genes selected from a group consisting of a gene encoding ICP6, a gene encoding TK, and a gene encoding UNG, wherein the site/sites of the tandem introduction of the cytokine genes is/are in at least one of the positions where the genes encoding ICP34.5, ICP47, ICP6, TK and UNG are deleted.

12. The genetically engineered herpes simplex virus according to claim 10 or 11, wherein the cytokines are selected from at least two of GM-CSF, IL-2, IL-12, TNF-α and IFN-γ.

13. The genetically engineered herpes simplex virus according to any one of claims 10-12, wherein the herpes simplex virus is herpes simplex virus type I.

14. A host cell comprising the recombinant herpes simplex virus of any one of claims 1-6 or the genetically engineered herpes simplex virus of any one of claims 10-13.

15. A virus stock solution comprising the recombinant herpes simplex virus of any one of claims 1-6 or the genetically engineered herpes simplex virus of any one of claims 10-13.

16. A pharmaceutical composition comprising the host cell of claim 14 or the virus stock solution of claim 15.

17. A use of the genetically engineered herpes simplex virus of any one of claims 10-13 or the host cell of claim 14 in the preparation of a medicament for treating a solid tumor.

18. The use according to claim 17, wherein the tumor is at least one selected from melanocytoma, brain glioma, head and neck tumor, liver cancer, lung cancer, colorectal cancer, renal cell carcinoma, gastric cancer, pancreatic cancer, lymphoma, bladder cancer, ovarian cancer, prostate cancer, and breast cancer.

19. A method for treating a tumor, comprising administering to a subject an effective amount of the recombinant herpes simplex virus of any one of claims 1-6 or the genetically engineered herpes simplex virus of any one of claims 10-13.

20. The method of claim 19, wherein the tumor is at least one selected from melanocytoma, brain glioma, head and neck tumor, liver cancer, lung cancer, colorectal cancer, renal cell carcinoma, gastric cancer, pancreatic cancer, lymphoma, bladder cancer, ovarian cancer, prostate cancer, and breast cancer.

21. The method of claim 19 or 20, wherein the herpes simplex virus is administered intratumorally via a catheter or injection.
